# EUROPEAN PATENT APPLICATION

(11) **EP 1 166 778 A2**
(43) Date of publication of application: **02.01.2002**
(21) Application number: 01305273.3
(22) Date of filing: 18.06.2001
(51) Int. Cl.: A61K 31/00, A61K 31/437, A61K 31/444, A61K 31/4985, A61K 31/55, A61K 31/438, A61K 45/06, A61P 37/00, A61P 1/00

(54) **The use of growth hormone scretagogues to treat systemic lupus erythematosus and inflammatory bowel disease**

(30) Priority: 19.06.2000 US 212521 P
(71) Applicant: Pfizer Products Inc., Groton, Connecticut 06340 (US)
(72) Inventor: Busch, Frank R. Pfizer Global Research and Dev., Groton, Connencticut 06340 (US); Lefker, Bruce A. Pfizer Global Research and Dev., Groton, Connencticut 06340 (US); Pan, Lydia C. Pfizer Global Research and Dev., Groton, Connencticut 06340 (US)
(74) Representative: Ruddock, Keith Stephen

(57) **Abstract**

This invention is directed to methods for treating systemic lupus erythematosus and/or inflammatory bowel disease such as Crohn's disease or ulcerative colitis in a patient which comprise administering a growth hormone secretagogue (GHS), prodrug thereof or a pharmaceutically acceptable salt of said GHS or said prodrug. More particularly, the present invention provides such methods wherein the GHS is a compound of Formula I: 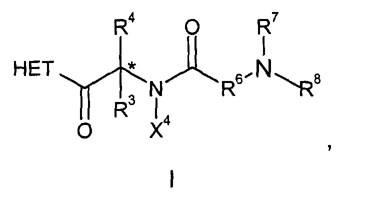 or a prodrug thereof or a pharmaceutically acceptable salt of said GHS or said prodrug. This invention is also directed to combinations of a GHS and a second therapeutic agent, where said second therapeutic agent is known to be beneficial in the treatment of systemic lupus erythematosus and/or inflammatory bowel disease such as Crohn's disease or ulcerative colitis, to kits and pharmaceutical compositions comprising such a combination and to methods of treating systemic lupus erythematosus and/or inflammatory bowel disease such as Crohn's disease or ulcerative colitis using such combinations, pharmaceutical compositions and kits.

## Description

### FIELD OF THE INVENTION

The present invention provides methods of using growth hormone secretagogues, prodrugs thereof and pharmaceutically acceptable salts of said secretagogues and said prodrugs to treat systemic lupus erythematosus, Crohn's disease, inflammatory bowel disease (IBD) and ulcerative colitis. More specifically, the present invention provides such methods wherein the growth hormone secretagogues are certain compounds of Formula I below. This invention also provides combinations comprising a growth hormone secretagogue and a second therapeutic agent selected from methotrexate, dapsone, a glucocorticoid or an antimalarial. The invention also provides pharmaceutical compositions and kits comprising such combinations and methods of using such combinations, pharmaceutical compositions and kits in the treatment of systemic lupus erythematosus, Crohn's disease, IBD and ulcerative colitis.

### BACKGROUND OF THE INVENTION

Systemic Lupus Erythematosus (SLE), also known as disseminated lupus erythematosus or, simply, lupus, is a chronic, usually life-long, potentially fatal autoimmune disease characterized by unpredictable exacerbations and remissions with protean clinical manifestations. SLE is also characterized by immune dysregulation resulting in the production of antinuclear antibodies (ANA), generation of circulating immune complexes, and activation of the complement system. In SLE there is a predilection for clinical involvement of the joints, skin, kidney, brain, serosa, lung, heart and gastrointestinal tract. The pathologic hallmark of the disease is recurrent, widespread, and diverse vascular lesions. Women and minorities are disproportionately affected and SLE is most common in women of child-bearing age although it has been reported in both extremes of life (e.g. diagnosed in infants and in the tenth decade of life). The number of persons suffering from SLE in the United States is reported variously from about 500,000 to about 2,000,000. (H. Michael Belmont, Clinical Overview of Lupus, http://cerebel.com/lupus/overview.html.)

The prognosis for patients with SLE has greatly improved over the last few decades with at least 80-90% of all patients surviving ten years. Thereafter, life expectancy approximates that of age matched controls. This improvement reflects the general advancements in health care (e.g. dialysis, antibiotics, antihypertensives, newer immunosuppressives with more favorable efficacy to toxicity ration) but also the specialized care available for patients with SLE. (Belmont, *ibid.*)

SLE is a complex disorder affecting a predominately young population and shares similarities with HIV infection as regards the propensity for multiple organ involvement, potentially life-threatening episodes, and need for sophisticated monitoring. (Belmont, *ibid.)*

SLE is not a rare disorder. Although reported at both extremes of life (e.g., diagnosed in infants and in the tenth decade of life), chiefly it affects women of child bearing age. Among children, SLE occurs three times more commonly in females than in males. In the 60% of SLE patients who experience onset of their disease between puberty and the fourth decade of life the female to male ratio is 9:1. Thereafter, the female preponderance again falls to that observed in prepubescents. (Belmont, *ibid.*)

The etiology of SLE remains unknown. A genetic predisposition, sex hormones, and environmental trigger(s) likely result in the disordered immune response that typifies the disease. (Belmont, *ibid*.)

A role for genetics is suggested by the increased percentage of two histocompatibility antigens in patients with SLE, HLA-DR2 and HLA-DR3. In addition, there is an increased frequency of the extended haplotype HLA-A1, B8, DR3. The role for heredity is further supported by the concordance for this illness among monozygotic twins. The polygenic nature, however, of this genetic predisposition as well as the contribution of environmental factors is suggested by the only moderate concordance rate which is reported to be between 25 and 60%. (Belmont, *ibid*.)

The origin of autoantibody production in SLE is unclear but a role has been suggested for an antigen driven process, spontaneous B-cell hyper-responsiveness, or impaired immune regulation. Regardless of the etiology of autoantibody production, SLE is associated with the impaired clearance of circulating immune complexes secondary to decreased CR1 expression, defective Fc receptor function, or deficiencies of early complement components such as C4A. (Belmont, *ibid.*) It has been suggested that the apoptosis process is atypical in the lupus patient leading to the increased production of autoantibodies including antiphospholipid antibodies. (L. Casciola-Rosen et al., Proc. Natl. Acad. Sci. USA, **93**, 1996, 1624-1629.

The health status of a patient with SLE is related not only to disease activity, but to the damage that results from recurrent episodes of disease flare (e.g., deforming arthropathy, shrinking lung, end stage renal disease, organic mental syndrome, etc.), as well as the adverse effects of treatment (e.g., avascular necrosis of bone, infections, precocious atherosclerosis, etc.). (H. Michael Belmont, Clinical Overview of Lupus, http://cerebel.com/lupus/overview.html.)

Current therapy for SLE consists of treatment with antimalarials, methotrexate, dapsone, corticosteroids and/or glucocorticoids. These treatments suffer from the drawback that the disease is not adequately controlled and that each of the current treatments have known serious side effects. For example, long term corticosteroid use can lead to osteoporosis, high blood pressure, arterial damage, increased risk of infection and cataracts. (http://www.nih.gov/niams/healthinfo/).

Inflammatory Bowel Disease is a term used to collectively describe Crohn's disease and ulcerative colitis, both of which cause similar symptoms, particularly inflammation of the intestines.

Crohn's disease is a debilitating multisystem disorder which is characterized by inflammation of the bowel. The disease usually begins during adolescence or early adulthood and often leads to catabolism. Generally, patients suffering from Crohn's disease are treated with immunosuppressive and antiflammatory drugs that often have severe side effects which may enhance the catabolic process. High protein diets have been partially successful in counteracting the effects of the disease. It has been reported that growth homrone and insulin-like growth factor I⁴ counteract the catabolic process of the disease and reduce morbidity. See Slonim et al., New England Journal of Medicine, 2000, **342**, 1633-1637. Crohn's disease and ulcerative colitis are currently treated by administering prednisone, sulfasalazine (which is sold in a commercial formulation as Azulfidine® ), mesalamine (which is variously sold in commercial formulations as, *inter alia,* Asacol® , Pentasa® and Rowasa® ) and olsalazine (which is sold in a commercial formulation as Dipentum® ).

Ulcerative colitis is a condition characterized by inflammation of the lining of the colon and/or rectum. The inflammation may be of long or short duration and varies in intensity. Remission, the period between flare-ups, may last for a few days to many years. Patients generally experience bloody diarrhea, rectal bleeding, abdominal pain or cramping and/or and urgent need to go to the bathroom. The condition is a life-long problem which has no cure short of removal of the colon. The periods of remission may be extended by treatment with prednisone, sulfasalazine (which is sold in a commercial formulation as Azulfidine® ), mesalamine (which is variously sold in commercial formulations as, *inter alia,* Asacol® , Pentasa® and Rowasa® ) and olsalazine (which is sold in a commercial formulation as Dipentum® ). (http://www.living-better.com/2010.html). Overexpression of bovine growth hormone in transgenic mice accelerates recovery and mucosal healing after experimentally induced colitis. See Williams et al., Gastroenterology 2000, **118**, A556.

Growth hormone (GH), which is secreted from the pituitary, stimulates growth of all tissues of the body that are capable of growing. In addition, growth hormone is known to have the following basic effects on the metabolic processes of the body: (1) increased rate of protein synthesis in all cells of the body; (2) decreased rate of carbohydrate utilization in cells of the body; and (3) increased mobilization of free fatty acids and use of fatty acids for energy.

GH receptors are found throughout the stomach, small intestine and colon. GH has a proliferative effect on intestinal crypt cells. Pair-fed transgenic mice overexpressing growth hormone show increases in small bowel weight and length and a 50% - 100% increase in mucosal mass with increased villus height and crypt depth, particularly in the proximal bowel. GH increases absorption or transport of water, sodium chloride, amino acids and calcium in the gut and increases enteroendocrine secretion in animals. See Shulman, Endocrine 2000, **12(2):** 147-152. In preliminary clinical trials of short bowel syndrome, GH in combination with glutamine and dietary supplementation improved fractional protein absorption and decreased stool output. See Byrne et al., Ann Surg 1995, **222:** 243-0255. High dose GH therapy improves survival in patients with severe hemorrhage from gastric stress ulcers. See Winawer et al., Arch Int Med 1975 **135:**569-572.

Various ways are known to release growth hormone (see Recent Progress in Hormone Research, vol. 52, pp. 215-245 (1997); and Front Horm Res. Basel, Karger, vol. 24, pp. 152-175 (1999)). For example, chemicals such as arginine, L-3,4-dihydroxyphenylalanine (L-DOPA), glucagon, vasopressin, and insulin induced hypoglycemia, as well as activities such as sleep and exercise, indirectly cause growth hormone to be released from the pituitary by acting in some fashion on the hypothalmus perhaps either to decrease somatostatin secretion or to increase the secretion of the known growth hormone secretagogue, growth hormone releasing factor (GRF), or the endogenous growth hormone-releasing hormone, ghrelin (see Nature, vol. 402, pp. 656-660 (9 December 1999), or all of these.

In cases where increased levels of growth hormone were desired, the problem was generally solved by providing exogenous growth hormone or by administering GRF, IGF-I or a peptidyl compound which stimulated growth hormone production and/or release. In any case, the peptidyl nature of the compound necessitated that it be administered by injection. Initially, the source of growth hormone was the extraction of the pituitary glands of cadavers. This resulted in a very expensive product and carried with it the risk that a disease associated with the source of the pituitary gland could be transmitted to the recipient of the growth hormone. Recombinant growth hormone has become available which, while no longer carrying any risk of disease transmission, is still a very expensive product which must be given by injection or by a nasal spray. In addition, administration of exogenous growth hormone may result in side-effects, including edema, and does not correlate with the pulsatile release seen in the endogenous release of growth hormone.

Certain compounds have been developed which stimulate the release of endogenous growth hormone. Peptides which are known to stimulate the release of endogenous growth hormone include growth hormone releasing hormone, the growth hormone releasing peptides, GHRP-6 and GHRP-1 (described in U.S. Patent No. 4,411,890; International Patent Application, Publication No. WO 89/07110; and International Patent Application, Publication No. WO 89/07111), and GHRP-2 (described in International Patent Application, Publication No. WO 93/04081), as well as hexarelin (J. Endocrinol. Invest., 15 (Suppl. 4): 45 (1992)). Other compounds possessing growth hormone secretagogue activity are disclosed in the following International Patent Applications (listed by Publication Nos.), issued U.S. Patents or published European Patent Applications: WO 98/46569, WO 98/51687, WO 96/38471, WO 96/35713, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, W097/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

In addition, the following growth hormone secretagogues are known in the art: MK-0677 (Merck); NN703 (Novo Nordisk); L-162752 and L-163022 (Merck); hexarelin (Pharmacia & Upjohn); GPA-748 (KP102, GHRP-2) (American Home Products); ipamorelin (Novo Nordisk); and LY444711 (Eli Lilly). The following agents that stimulate GH release via GHRH/GRF receptor (including GHRH/GRF derivatives, analogs and mimetics) are known in the art: Geref (Ares/Serono); GHRH (1-44) (BioNebraska); Somatorelin (GRF 1-44) (Fujisawa/ICN); and ThGRF (Theratechnologies).

Endocrine Reviews 18(5): 621-645 (1997) provides an overview of peptidomimetic regulation of growth hormone secretion by growth hormone secretagogues. Horm. Res. 1999; 51 (suppl 3):16-20 (1999), examines the clinical and experimental effects of growth hormone secretagogues on various organ systems.

As is well known to those skilled in the art, the known and potential uses of growth hormone are varied and multitudinous. See "Human Growth Hormone," Strobel and Thomas, Pharmacological Reviews, 46, pg. 1-34 (1994). Also, these varied uses of growth hormone are summarized in International Patent Application, Publication Number WO 97/24369.

International Patent Applications, Publication Numbers WO 97/24369 and WO 98/58947 disclose that certain growth hormone secretagogues are useful for the treatment and prevention of osteoporosis, congestive heart failure, frailty associated with aging, obesity; accelerating bone fracture repair, attenuating protein catabolic response after a major operation, reducing cachexia and protein loss due to chronic illness, accelerating wound healing or accelerating the recovery of burn patients or patients having undergone major surgery; improving muscle strength, mobility, maintenance of skin thickness, metabolic homeostasis or renal homeostasis. Published European patent application 0995748 discloses that certain dipeptide growth hormone secretagogues are useful for the treatment or prevention of musculoskeletal frailty, including osteoporosis.

The administration of a growth hormone secretagogue is also known to enhance the quality of sleep, which is disclosed in International Patent Application Publication Number WO 97/24369. Commonly assigned U.S. nonprovisional patent application 09/290985, filed 13 April 1999, discloses pharmaceutical compositions comprising certain β₃ adrenergic agonists and growth hormone secretagogues or growth hormone, and their use for treating diabetes, obesity, hyperglycemia, frailty associated with obesity or frailty associated with aging, and for enhancing the quality of sleep in a mammal. International Patent Application Publication Number WO 98/58949 discloses the treatment of insulin resistance with certain growth hormone secretagogues.

International Patent Application Publication Number WO 00/12407 discloses that a growth hormone secretagogue is useful for enhancing the return of patients to independent living status following acute deconditioning such as that which may result from immobilization, surgery, or major injury such as hip fracture.

Journal of Orthopaedic Research 15:519:527 (1997) discloses that a growth hormone secretagogue, MK-0677, elevated levels of serum insulin-like growth factor-1, which in turn increased the size and strength of the quadriceps muscle in canines during remobilization.

J. Bone Miner. Res. 1998, 12: 1158-1166 discloses that treatment with the oral growth hormone secretagogue, MK-0677, increases markers of bone formation and bone resorption in obese young males.

Bone 23(5) (Supplement), Abstract F235 from ASBMR/IBMS Joint Meeting (November 1998), discloses that the growth hormone secretagogues, GHRP-6 and Ipamorelin (IPA) have the capacity to increase bone mass in adult female rats.

U.S. Patent Number 6,043,026 (28 March 2000) discloses that the combination of an estrogen receptor modulator and a growth hormone secretagogue is useful in the treatment or prevention of diseases involving bone resorption, especially osteoporosis.

R. Bross et al., J. Clin. Endocrinol. Metab., 84:3420-3430 (1999) discusses the potential use of human growth hormone supplementation for the treatment of aging-associated sarcopenia.

### SUMMARY OF THE INVENTION

This invention is directed to methods of treating systemic lupus erythematosus in a patient which comprises administering to the patient a systemic lupus erythematosus treating effective amount of a growth hormone secretagogue (GHS), a prodrug thereof or a pharmaceutically acceptable salt of said GHS or said prodrug.

This invention is also directed to methods of treating inflammatory bowel disease such as Crohn's disease and ulcerative colitis in a patient which comprises administering to the patient an inflammatory bowel disease treating effective amount of a growth hormone secretagogue (GHS), a prodrug thereof or a pharmaceutically acceptable salt of said GHS or said prodrug.

The GHS may be peptidyl or non-peptidyl in nature, however, the use of an orally active GHS is preferred. In addition, it is preferred that the GHS induce or amplify a pulsatile release of endogenous growth hormone.

This invention is also directed to combinations comprising a GHS, a prodrug thereof or a pharmaceutically acceptable salt of said GHS or said prodrug and a second therapeutic agent wherein said second therapeutic agent is known to be beneficial in the treatment of systemic lupus erythematosus. Preferred such agents include, but are not limited to, methotrexate, dapsone, a glucocorticoid or an antimalarial, a prodrug of said methotrexate, dapsone, glucocorticoid or antimalarial or a pharmaceutically acceptable salt thereof or of said prodrug. This invention is also directed to pharmaceutical compositions comprising such combinations and a pharmaceutically acceptable carrier, vehicle or diluent.

This invention is also directed to a kit comprising:
a) a first unit dosage form comprising a GHS, a prodrug thereof or a pharmaceutically acceptable salt of said GHS or said prodrug and a pharmaceutically acceptable carrier, vehicle or diluent;
b) a second unit dosage form comprising methotrexate, dapsone, a glucocorticoid or an antimalarial, a prodrug thereof or a pharmaceutically acceptable salt of methotrexate, dapsone, glucocorticoid or antimalarial or said prodrug and a pharmaceutically acceptable carrier, vehicle or diluent; and
c) a container.

This invention is also directed to methods of treating systemic lupus erythematosus in a patient which comprises administering to the patient
a) a pharmaceutical composition comprising a GHS, a prodrug thereof or a pharmaceutically acceptable salt of said GHS or said prodrug; and methotrexate, dapsone, a glucocorticoid or an antimalarial, a prodrug thereof or a pharmaceutically acceptable salt of methotrexate, dapsone, glucocorticoid or antimalarial or said prodrug; and a pharmaceutically acceptable carrier, vehicle or diluent;
b) a GHS, a prodrug thereof or a pharmaceutically acceptable salt of said GHS or said prodrug or a pharmaceutical composition thereof; and methotrexate, dapsone, a glucocorticoid or an antimalarial, a prodrug thereof or a pharmaceutically acceptable salt of said methotrexate, dapsone, glucocorticoid or antimalarial or said prodrug or a pharmaceutical composition thereof; or
c) a kit as described herein. This invention thus includes methods whereby a fixed combination is administered and methods whereby the individual components of the combination are administered separately.

This invention is also directed to combinations comprising a GHS, a prodrug thereof or a pharmaceutically acceptable salt of said GHS or said prodrug and a second therapeutic agent wherein said second therapeutic agent is known to be beneficial in the treatment of inflammatory bowel disease such as Crohn's disease or ulcerative colitis. Preferred such agents include, but are not limited to, prednisone, sulfasalazine, mesalamine and olsalazine, a prodrug thereof or a pharmaceutically acceptable salt of said agent or said prodrug and a pharmaceutically acceptable carrier, vehicle or diluent.

This invention is also directed to pharmaceutical compositions comprising such combinations and a pharmaceutically acceptable carrier, vehicle or diluent.

This invention is also directed to a kit comprising:
a) a first unit dosage form comprising a GHS, a prodrug thereof or a pharmaceutically acceptable salt of said GHS or said prodrug and a pharmaceutically acceptable carrier, vehicle or diluent;
b) a second unit dosage form comprising an agent selected from prednisone, sulfasalazine, mesalamine and olsalazine, a prodrug thereof or a pharmaceutically acceptable salt of said agent or said prodrug and a pharmaceutically acceptable carrier, vehicle or diluent; and
c) a container.

This invention is also directed to methods of treating inflammatory bowel disease such as Crohn's disease or ulcerative colitis in a patient which comprises administering to the patient
a) a pharmaceutical composition comprising a GHS, a prodrug thereof or a pharmaceutically acceptable salt of said GHS or said prodrug; and an agent selected from prednisone, sulfasalazine, mesalamine and olsalazine, a prodrug thereof or a pharmaceutically acceptable salt of said agent or said prodrug and a pharmaceutically acceptable carrier, vehicle or diluent;
b) a GHS, a prodrug thereof or a pharmaceutically acceptable salt of said GHS or said prodrug or a pharmaceutical composition thereof; and an agent selected from prednisone, sulfasalazine, mesalamine and olsalazine, a prodrug thereof or a pharmaceutically acceptable salt of said agent or said prodrug or a pharmaceutical composition thereof; or
c) a kit as described herein. This invention thus includes methods whereby a fixed combination is administered and methods whereby the individual components of the combination are administered separately.

In the combinations, methods, pharmaceutical compositions and kits of this invention, it is preferred that the GHS is a compound of the Formula I: 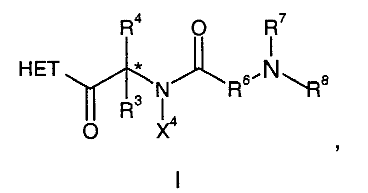 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug,
wherein:
HET is a heterocyclic moiety selected from the group consisting of 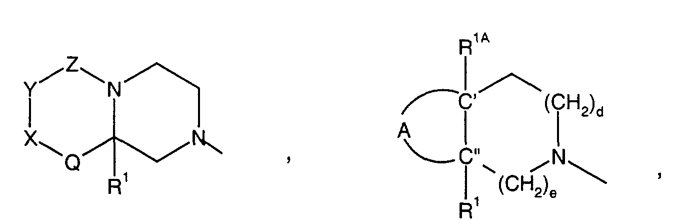 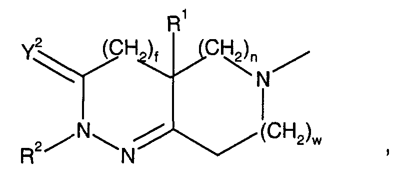 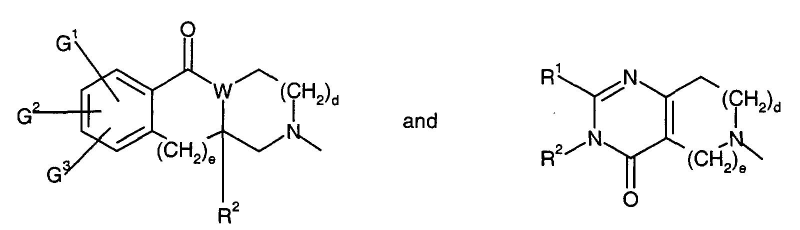
d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
   -NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-,-O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino, -(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
   Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
   q is 0, 1, 2, 3 or 4;
   t is 0,1, 2 or 3;
   said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxy, -C(O)OX⁶, -C(O)N(X⁶)(X⁶), -N(X⁶)(X⁶), - S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
   where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
   X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 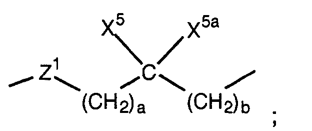
   where a and b are each independently 0, 1, 2 or 3;
   X⁵ and X^{5a} are each independently selected from the group consisting of hydrogen, CF₃, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
   or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
   or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
   or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
   Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of formula I;
   E is -O-, -S-, -CH=CH- or an aromatic moiety selected from 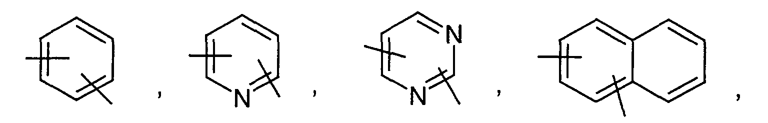 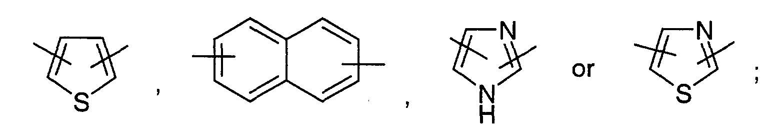 said aromatic moiety in the definition of E optionally substituted with up to three halo, hydroxy, -N(R^{c})(R^{c}), (C₁-C₆)alkyl or (C₁-C₆)alkoxy;
   R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl,
      -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
   R^{c}, for each occurrence, is independently hydrogen or (C₁-C₆)alkyl;
   a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
   where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
   A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
   where X¹¹ is hydrogen or optionally substituted (C₁-C₆)alkyl;
      the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
   X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
   or X¹¹ and X¹² are taken together to form -(CH₂)ᵣ-L¹-(CH₂)ᵣ-;
      L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1 H-tetrazol-5-yl; or
   when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
   X⁷ is hydrogen or (C₁-C₆)alkyl optionally substituted with hydroxy;
m for each occurrence is independently 0, 1 or 2;
   with the provisos that:
   1) X⁶ and X¹² cannot be hydrogen when attached to C(O) or S(O)₂ in the form C(O)X⁶, C(O)X¹², S(O)₂X⁶ or S(O)₂X¹²; and
   2) when R⁶ is a bond then L is N(X²) and each r in the definition -(CH₂)ᵣ-L-(CH₂)ᵣ- is independently 2 or 3. It is preferred that the GHS is a compound of the formula 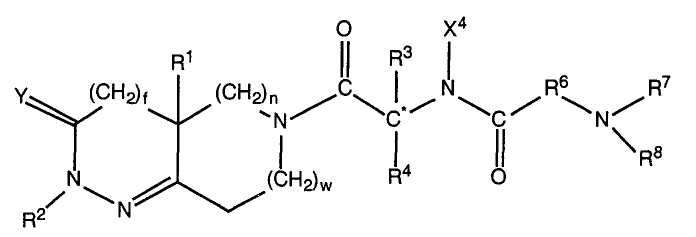 or a racemic-diastereomeric mixture or an optical isomer of said compound or a pharmaceutically-acceptable salt or prodrug thereof,
   wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro; Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
   q is 0, 1, 2, 3 or 4;
   t is 0, 1, 2 or 3;
   said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ or -(C₁-C₅)alkyl-X¹-(C₁-C₅)alkyl-(C₃-C₇)cycloalkyl;
   where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³; X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 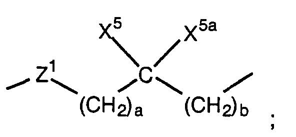
   where a and b are independently 0, 1, 2 or 3;
   X⁵ and X^{5a} are each independently selected from the group consisting of hydrogen, trifluoromethyl, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
   where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8- membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, optionally having 1 to 4 heteroatoms independently selected from the group consisting of nitorgen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
   A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
      where X¹¹ is hydrogen or optionally substituted (C₁-C₆)alkyl;
      the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
   X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
   or X¹¹ and X¹² are taken together to form -(CH₂)ᵣ-L¹-(CH₂)ᵣ-;
      where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂, -S(O)ₘ(C₁-C₆)alkyl, carboxylate (C₁-C₄)alkyl ester, or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
   X⁷ is hydrogen or (C₁-C₆)alkyl optionally substituted with hydroxyl; and
m for each occurrence is independently 0, 1 or 2;
   with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

In the combinations, pharmaceutical compositions, methods and kits of this invention, it is even more preferred that said GHS is 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl]-2-oxo-ethyl)-2-methyl-propionamide; 2-amino-N-[2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide; or 2-amino-N-(1-(R)-(2,4-difluoro-benzyloxymethyl)-2-oxo-2-(3-oxo-3a-(R)-pyridin-2-ylmethyl)-2-(2,2,2-trifluoroethyl)-2,3,3a,4,6,7-hexahydro-pyrazolo[4,3-c]pyridin-5-yl)-ethyl)-2-methyl-propionamide, or a prodrug thereof or a pharmaceutically acceptable salt thereof or of said prodrug.

In the combinations, pharmaceutical compositions, methods and kits of this invention, it is still more especially preferred that the L-tartrate salt of 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl]-2-oxo-ethyl)-2-methyl-propionamide; the L-tartrate salt of 2-amino-N-(2-(3a(R)-benzyl-2-methyl-3-oxo-2,3, 3a, 4,6,7-hexahydropyrazolo-[4,3-c]pyridin-5-yl)-1 (R)-benzyloxymethyl-2-oxo-ethyl)-isobutyramide; or the L-tartrate salt of 2-amino-N-(1-(R)-(2,4-difluoro-benzyloxymethyl)-2-oxo-2-(3-oxo-3a-(R)-pyridin-2-yl)-2-(2,2,2-trifluoro-ethyl)-2,3,3a,4,6,7-hexahydro-pyrazolo-[4,3-c]pyridin-5-yl)-ethyl)-2-methyl-propionamide is used.

This invention is also directed to methods comprising administering a recombinant growth hormone or an additional GHS selected from the group consisting of GHRP-6, GHRP-1, GHRP-2, growth hormone releasing factor and an analog of growth hormone releasing factor in addition to said GHS.

In the combinations of this invention it is preferred that said glucocorticoid is prednisone, betamethasone dipropionate, clobetasol, diflorasone diacetate, halobetasol propionate, amcinonide, desoximetasone, fluocinonide, halcinonide, betamethasone valerate, triamcinolone acetate, fluocinolone acetonide, flurandrenolide, hydrocortisone valerate, triamcinolone acetonide, hydrocortisone butyrate, alclometasone dipropionate, desonide, mometasone furoate, dexamethasone, hydrocortisone or methylprednisolone acetate. Also preferred in this invention are combinations wherein said antimalarial is chloroquine, hydroxychloroquine, quinacrine or quinine.

The term "pharmaceutically acceptable" means that a substance or mixture of substances must be compatible with the other ingredients of a formulation and not deleterious to a patient.

The term "treating", "treat" or "treatment" as used herein includes curative, preventative (e.g., prophylactic) and palliative treatment.

The terms "patient" and "subject" are used interchangeably and refer to animals, particularly mammals such as dogs, cats, cattle, horses, sheep and humans. Particularly preferred patients and subjects are humans, including males and females.

The term "therapeutically effective amount" means an amount of a GHS that ameliorates, attenuates, or eliminates a particular disease or condition associated with growth hormone secretion and/or production, or prevents or delays the onset of a disease or condition associated with growth hormone secretion and/or production.

The parenthetical negative or positive sign used herein in the nomenclature denotes the direction plane polarized light is rotated by the particular stereoisomer.

The subject invention also includes combinations, pharmaceutical compositions, methods and kits comprising isotopically-labeled compounds, which are identical to the compounds described hereinabove, but for the fact that one or more atoms are replaced by an atom having an atomic mass or mass number different from the atomic mass or mass number usually found in nature. Examples of isotopes that can be incorporated into compounds used in the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds used in the present invention, prodrugs thereof, and pharmaceutically acceptable salts of said compounds or of said prodrugs which contain the aforementioned isotopes and/or other isotopes of other atoms are within the scope of this invention. Certain isotopically-labeled compounds of the present invention, for example those into which racioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled compounds used in this invention and prodrugs thereof can generally be prepared by carrying out the procedures disclosed in the Schemes and /or in the Examples and Preparations described in the patents and applications which are incorporated herein by reference, by substituting a readily available isotopically labeled reagent for a non-isotopically labeled reagent.

Other features and advantages will be apparent from the description and claims which describe the invention.

### DETAILED DESCRIPTION OF THE INVENTION

The present invention is directed to the use of a compound, which has the ability to stimulate or amplify the release of natural or endogenous growth hormone, for treating systemic lupus erythematosus. In particular, the present invention provides a method for treating systemic lupus erythematosus comprising the administration of a GHS, a prodrug thereof or a pharmaceutically acceptable salt of said GHS or of said prodrug. The present invention also provides a method for treating IBD such as Crohn's disease or ulcerative colitis comprising the administration of a GHS, a prodrug thereof or a pharmaceutically acceptable salt of said GHS or of said prodrug.

By the term "growth hormone secretagogue" is meant any exogenously administered compound or agent that directly or indirectly stimulates or increases the endogenous release of growth hormone, growth hormone-releasing hormone or somatostatin in an animal, in particular, a human.

In the treatment of systemic lupus erythematosus, the GHS may be used alone or in combination with other GHSs or with other agents which are known to be beneficial for the treatment of systemic lupus erythematosus. The GHS and the other agent may be coadministered, either in concomitant therapy or in a fixed combination. For example, the GHS may be administered in combination with methotrexate, dapsone, a glucocorticoid or an antimalarial.

In the treatment of IBD such as Crohn's disease or ulcerative colitis, the GHS may be used alone or in combination with other GHSs or with other agents which are known to be beneficial for the treatment of IBD such as Crohn's disease or ulcerative colitis. The GHS and the other agent may be coadministered, either in concomitant therapy or in a fixed combination. For example, the GHS may be administered in combination with prednisone, sulfasalazine, mesalamine or olsalazine.

Representative GHSs and a full description of procedures for preparing those GHSs, are disclosed in the following International Patent Applications (listed by Publication Nos.), issued U.S. patents and published European patent applications, each of which is incorporated herein by reference: WO 98/46569, WO 98/51687, WO 96/38471, WO 96/35713, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A representative first group of GHSs is set forth in International Patent Application, Publication No. WO 97/24369, as compounds having the structural formula below, which is designated herein as Formula II: 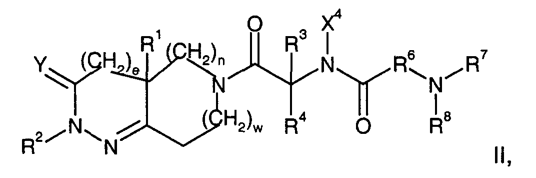 wherein the various substituents are as defined in WO 97/24369. Said compounds are prepared as disclosed therein.

2-Amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide, having the following structure: 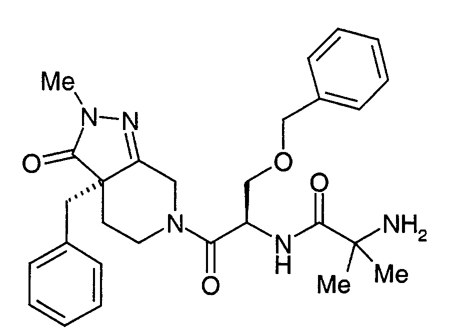 and 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide, having the following structure: 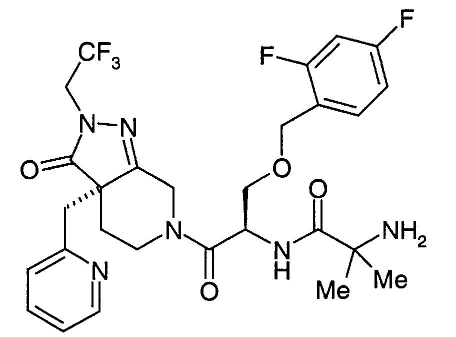 are both within the scope of the disclosure of International Patent Application, Publication Number WO 97/24369.

A representative second group of GHSs is set forth in International Patent Application, Publication No. WO 98/58947, as compounds having the structural formula below, which is designated herein as Formula III: 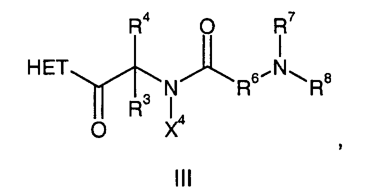 wherein the various substituents are as defined in WO 98/58947. Said compounds are prepared as disclosed therein or as described herein.

A preferred compound within this second group which may be employed in the present invention is identified as having the following name and structure: 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, 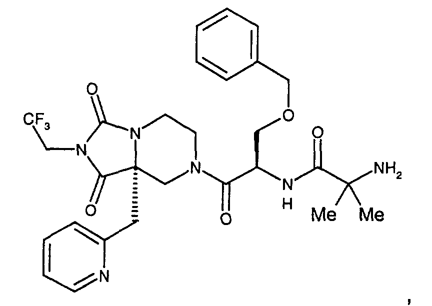 This compound is within the scope of the disclosure of International Patent Application, Publication No. WO 98/58947, and may be prepared as described in Examples Five and Six therein.

A representative third group of GHSs is set forth in Published European patent application 0995748, which discloses certain dipeptide GHSs of Formula III, as designated above, and their use for the treatment or prevention of musculoskeletal fraility including osteoporosis.

A representative fourth group of GHSs is set forth in U.S. Patent No. 5,206,235, as having the following structure: 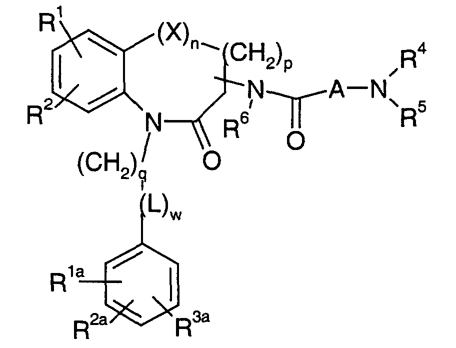 wherein the various substituents are as defined in U.S. Patent No. 5,206,235. Said compounds are prepared as disclosed therein.

Preferred compounds within this fourth group are identified as having the following structures: 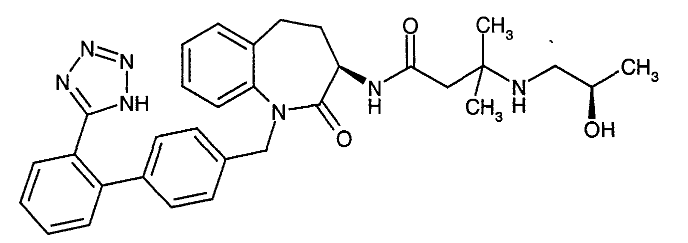 or

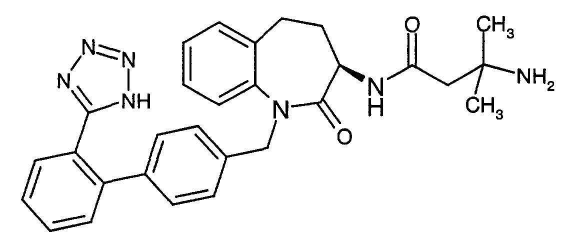

A representative fifth group of GHSs is set forth in U.S. Patent No. 5,283,241, as having the following structural formula: 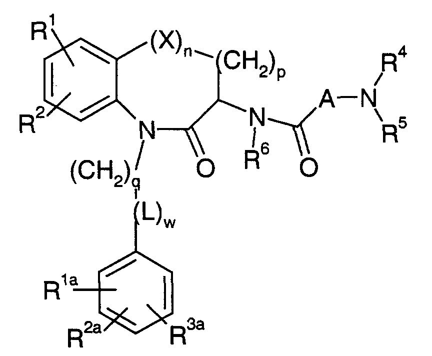 wherein the various substituents are as defined in U.S. Patent 5,283,241. Said compounds are prepared as disclosed therein.

A representative sixth group of GHSs is disclosed in International Patent Application, Publication No. WO 97/41879, as compounds having the following structural formulas: 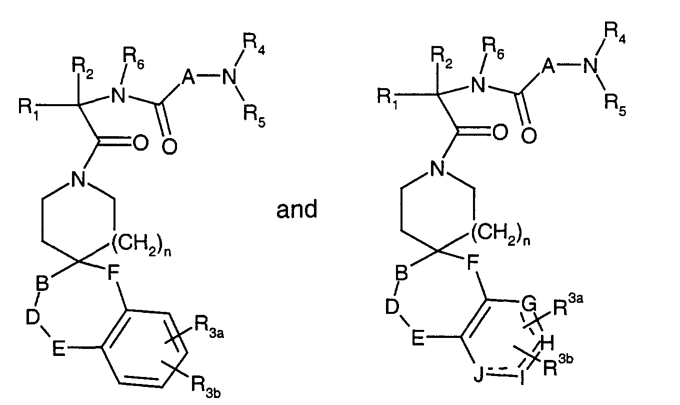 wherein the various substituents are as defined in WO97/41879. Said compounds are prepared as disclosed therein.

Preferred compounds within this sixth group which may be employed in the present invention are identified as having the following structure: 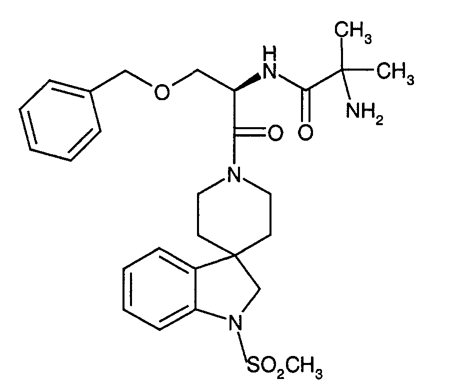 and pharmaceutically acceptable salts thereof, in particular, the methanesulfonate salt.

A representative seventh group of GHSs is disclosed in U.S. Patent No. 5,492,916, as being compounds of the following structural formula: 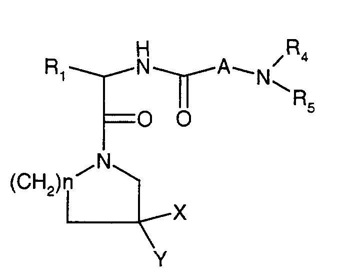 wherein the various substituents are as defined in U.S. Patent No. 5,492,916. Said compounds are prepared as disclosed therein.

All of these compounds may be prepared by procedures disclosed in these publications. Full descriptions of the preparation of the GHSs which may be employed in the present invention may be found in the art, particularly in the references cited herein.

As set forth hereinabove, a second therapeutic agent may be used in conjunction with the GHS in the combinations, pharmaceutical compositions, kits and methods of this invention. When the second therapeutic agent is a glucocorticoid, suitable glucocorticoids are prednisone, betamethasone dipropionate, clobetasol, diflorasone diacetate, halobetasol propionate, amcinonide, desoximetasone, fluocinonide, halcinonide, betamethasone valerate, triamcinolone acetate, fluocinolone acetonide, flurandrenolide, hydrocortisone valerate, triamcinolone acetonide, hydrocortisone butyrate, alclometasone dipropionate, desonide, mometasone furoate, dexamethasone, hydrocortisone or methylprednisolone acetate. Other glucocorticoids are also within the scope of the combinations of this invention.

When the second therapeutic agent is an antimalarial, suitable antimalarials include quinine, chloroquine, quinacrine and hydroxychloroquine. Other antimalarial agents are also within the scope of the combinations, pharmaceutical compositions, kits and methods of this invention.

The second therapeutic agents set forth herein, including methotrexate, dapsone, the glucocorticoids disclosed herein and the antimalarials disclosed herein, are all readily available or can be prepared as set forth in references which may be found in the Merck Index, 12^{th} Edition, Merck & Co., Whitehouse Station, New Jersey, 1996.

Mesalamine, also known as 5-amino-2-hydroxybenzoic acid, is prepared from 2-hydroxy-5-nitrobenzoic acid (Aldrich Chemical Company, P.O. Box 355, Milwaukee, Wisconsin 52301-9358) by reduction methods well known to those skilled in the art. A particularly useful process for effecting said reduction is by reacting said 2-hydroxy-5-nitrobenzoic acid with zinc dust and hydrochloric acid as described by Weil et al., Ber. **55B,** 2664 (1922).

Sulfasalazine may be prepared as described in United States Patent Number 2,396,145.

Olsalazine may be prepared as described in United States Patent Number 4,528,367.

Prednisone may be prepared as described in United States Patent Numbers 2,897,216; 2,837,464; or 3,134,718; or as described in Herzog et al., Tetrahedron, **18**, 581 (1962) or Nobile et al., JACS, **77**, 4184 (1955).

The expression "pharmaceutically acceptable salts" includes both pharmaceutically acceptable acid addition salts and pharmaceutically acceptable cationic salts, where appropriate. The expression "pharmaceutically-acceptable cationic salts" is intended to define but is not limited to such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, d-tartrate, I-tartrate, citrate, methanesulfonate (mesylate) and p-toluenesulfonate (tosylate) salts.

Pharmaceutically acceptable cationic salts of the compounds used in this invention may be readily prepared, where appropriate, by reacting the free acid form of said compound with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), and employing a solvent (e.g., ethyl acetate) from which the desired cationic salt precipitates, or can be otherwise isolated by concentration and/or addition of a non-solvent.

The acid addition salts of the compounds used in this invention may be readily prepared by reacting the free base form of said compound with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

In addition, the GHSs and other compounds which may be used in accordance with this invention, prodrugs thereof and pharmaceutically acceptable salts thereof or of said prodrugs, may occur as hydrates or solvates. Said hydrates and solvates are also within the scope of the invention.

The compounds used in the methods of the present invention may have at least one asymmetric center as noted, e.g., by the asterisk in the structural Formula I-A below. Additional asymmetric centers may be present in the compounds of Formula I depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, be included within the scope of the methods and combinations of the instant invention. In the case of the asymmetric center represented by the asterisk, it has been found that the absolute stereochemistry of the more active and thus more preferred isomer is shown in Formula I-A below: 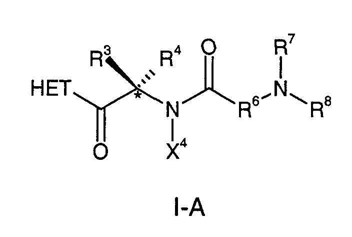 With the R⁴ substituent as hydrogen, the spatial configuration of the asymmetric center corresponds to that in a D-amino acid. In most cases this is also designated an R-configuration although this will vary according to the values of R³ and R⁴ used in making R- or S-stereochemical assignments.

Certain compounds within the scope of the combinations, pharmaceutical compositions, kits and methods of this invention may have the potential to exist in tautomeric forms. All tautomers of a compound of the present invention are within the scope of this invention. Also, for example, all keto-enol or imine-enamine forms of the compounds are included in this invention.

Those skilled in the art will recognize that the compound names contained herein may be based on a particular tautomer of a compound. While the name for only a particular tautomer may be used, it is intended that all tautomers are encompassed by the name of the particular tautomer and all tautomers are considered part of the present invention.

A GHS is a compound that, when administered to a patient, increases the production and/or secretion of growth hormone when compared with baseline plasma concentrations of growth hormone. Thus, to identify a GHS, one need simply measure the baseline plasma concentrations of growth hormone over a time period, typically one day, and compare the plasma concentrations of growth hormone after administration of a GHS with the baseline concentration over the time period. Various examples of GHSs are disclosed herein. It is contemplated that any GHS can be used in the present administration methods.

The identification of a compound as a "growth hormone secretagogue" which is able to directly or indirectly stimulate or increase the endogenous release of growth hormone in an animal may be readily determined without undue experimentation by methodology well known in the art, such as the assay described by Smith et al., Science, **260**, 1640-1643 (1993) (see text of Figure 2 therein). In a typical experiment, pituitary glands are aseptically removed from 150-200 g Wistar male rats and cultures of pituitary cells are prepared according to Cheng et al., Endocrinol., **124**, 2791-2798 (1989). The cells are treated with the subject compound and assayed for growth hormone secreting activity, as described by Cheng et al. (*ibid*.). In particular, the GHS activity of a compound which may be used in the present invention may be determined by this assay. Compounds which display GHS activity in this assay are useful in treating systemic lupus erytheamtosus and intestinal bowel disease as described herein.

This particular application of GHS provides unexpected benefits relative to the administration of exogenous growth hormone. In particular, the GHS enhances the normal pulsatile releases of endogenous growth hormone and thus is more likely to reproduce the natural pattern of endogenous growth hormone release (see J. Clin. Endocrinol. Metab. **81**: 4249-4257, 1996). GHSs which are orally active also have the benefit of being able to be administered orally, rather than just intravenously, intraperitoneally or subcutaneously.

In view of their use according to the present invention, the GHSs used in the present invention may be formulated into various pharmaceutical forms for administration purposes. A GHS may be administered, alone or in combination, by oral, parenteral (e.g., intramuscular, intraperitoneal, intravenous or subcutaneous injection, or implant), nasal, vaginal, rectal, sublingual, or topical routes of administration and can be formulated with pharmaceutically acceptable vehicles, diluents or carriers to provide dosage forms appropriate for each route of administration.

Solid dosage forms for oral administration include capsules, tablets, pills, powders and granules and for companion animals the solid dosage forms include an admixture with food and chewable forms. In such solid dosage forms, the compounds and combinations of this invention can be admixed with at least one inert pharmaceutically acceptable carrier, vehicle or diluent such as sucrose, lactose, or starch. Such dosage forms can also comprise, as is normal practice, additional substances other than such inert carriers, vehicles or diluents, e.g., lubricating agents such as magnesium stearate. In the case of capsules, tablets and pills, the dosage forms may also comprise buffering agents. Tablets and pills can additionally be prepared with enteric coatings. In the case of chewable forms, the dosage form may comprise flavoring agents and perfuming agents.

Liquid dosage forms for oral administration include pharmaceutically acceptable emulsions, solutions, suspensions, syrups, and elixirs containing inert vehicles, carriers or diluents commonly used in the art, such as water. Besides such inert vehicles, carriers or diluents, such compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, which may also be a prodrug or a pharmaceutically acceptable salt of a prodrug, excipients such as coca butter or a suppository wax. Compositions for nasal or sublingual administration are also prepared with standard excipients well known in the art.

The dosage of active ingredients in the compositions, methods and combinations of the present invention invention may be varied; however, it is necessary that the amount of the active ingredients be such that a suitable dosage form is obtained. The selected dosage depends upon the desired therapeutic effect, on the route of administration, and on the duration of the treatment. Generally, dosage levels of between 0.001 to 10 mg/kg of body weight daily are administered to humans and other animals, e.g., mammals, to obtain effective release of growth hormone. A preferred dosage range in humans is 0.01 to 5.0 mg/kg of body weight daily which can be administered as a single dose or divided into multiple doses.

A preferred dosage range in animals other than humans is 0.01 to 10.0 mg/kg of body weight daily which can be administered as a single dose or divided into multiple doses. A more preferred dosage range in animals other than humans is 0.1 to 5 mg/kg of body weight daily which can be administered as a single dose or divided into multiple doses.

Where the tartrate salt or other pharmaceutically acceptable salt of the above compounds is used in the present invention, the skilled person will be able to calculate effective dosage amounts by calculating the molecular weight of the salt form and performing simple stoichiometric ratios.

Also, the present invention includes within its scope the use of a GHS according to the present invention, alone or in combination with another GHS, such as those referenced herein, including the growth hormone releasing peptides GHRP-6 and GHRP-1 (described in U.S. Patent No. 4,411,890 and International Patent Applications, Publication Nos. WO 89/07110, WO 89/07111) and GHRP-2 (described in WO 93/04081) and B-HT920, as well as hexarelin and growth hormone releasing hormone (GHRH, also designated GRF) and its analogs, and growth hormone and its analogs, or in combination with other therapeutic agents, such as β-adrenergic agonists such as clonidine or serotonin 5HTD agonists such as sumatriptan, or agents which inhibit somatostatin or its release such as physostigmine and pyridostigmine. Preferably, the GHS may be used in combination with growth hormone releasing factor or an analog of growth hormone releasing factor.

In addition, the present invention includes within its scope the use of a pharmaceutical composition according to the present invention comprising, as an active ingredient, at least one GHS in association with a pharmaceutical carrier, vehicle or diluent.

It will be known to those skilled in the art that other compounds may be used in an effort to treat systemic lupus erythematosus. Combinations of these therapeutic agents, some of which have been mentioned herein, with a GHS will bring additional complementary, and potentially synergistic properties to enhance the desirable properties of these various therapeutic agents. Systemic lupus erythematosus treating agents other than those described herein are also within the scope of the combinations of this invention. In these combinations, the GHS and the other therapeutic agent(s) may be independently present in the dose ranges from 0.01 to 1 times the dose levels which are effective when these compounds and secretagogues are used singly.

It will also be known to those skilled in the art that other compounds may be used in an effort to treat IBD such as Crohn's disease and ulcerative colitis. Combinations of these therapeutic agents, some of which have been mentioned herein, with a GHS will bring additional complementary, and potentially synergistic properties to enhance the desirable properties of these various therapeutic agents. IBD treating agents other than those described herein are also within the scope of the combinations of this invention. In these combinations, the GHS and the other therapeutic agent(s) may be independently present in the dose ranges from 0.01 to 1 times the dose levels which are effective when these compounds and secretagogues are used singly.

Typically, the individual daily dosages for these combinations may range from about one-fifth of the minimally recommended clinical dosages to the maximum recommended levels for the entities when they are given singly. These dose ranges may be adjusted on a unit basis as necessary to permit divided daily dosage and, as noted above, the dose will vary depending on the nature and severity of the disease, weight of patient, special diets and other factors.

These combinations may be formulated into pharmaceutical compositions as known in the art and as discussed herein.

Since the present invention has an aspect that relates to treatment with a combination of active ingredients which may be administered separately, the invention also relates to combining separate pharmaceutical compositions in kit form. The kit comprises two separate pharmaceutical compositions: a GHS, a prodrug thereof or a pharmaceutically acceptable salt of said GHS or said prodrug; and a second therapeutic agent as described herein. The kit comprises a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of a second therapeutic agent as described herein can consist of one tablet or capsule while a daily dose of the GHS, prodrug thereof or pharmaceutically acceptable salt of said GHS or said prodrug can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The utility of the compounds described herein in the methods of the present invention are demonstrated by their activity in one or more of the assays which are described in International Application Publication Number WO97/24369.

It will be understood that the practice of the present invention encompasses all of the usual variations, adaptations or modifications as come within the scope of the following claims and their equivalents.

## Claims

1. The use of an effective amount of a growth hormone secretagogue (GHS), a prodrug thereof or a pharmaceutically acceptable salt of said GHS or of said prodrug for the manufacture of a medicament for the treatment of systemic lupus erythematosus or inflammatory bowel disease.

2. A use according to claim 1 wherein said GHS is a compound of the Formula I: 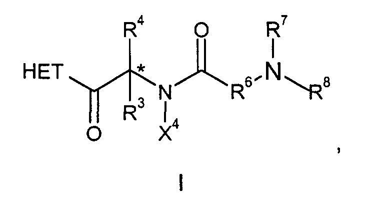 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug,
wherein:
HET is a heterocyclic moiety selected from the group consisting of 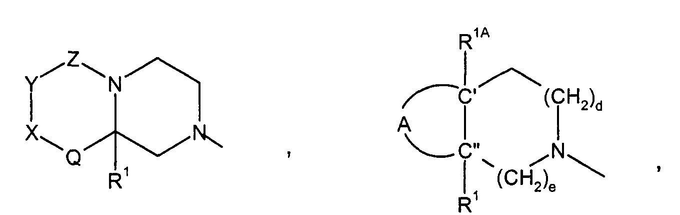 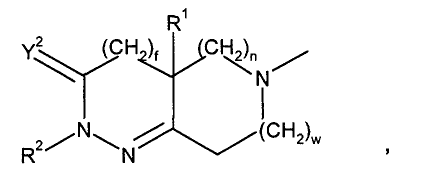 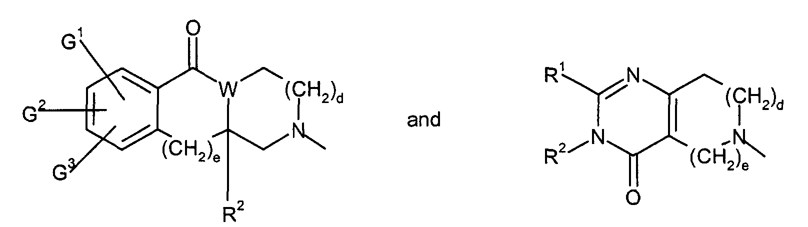
d is 0, 1 or 2;
e is 1 or 2;
f is 0 or 1 ;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
-NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino,-(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹,
-(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ᵣA¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxy, -C(O)OX⁶, -C(O)N(X⁶)(X⁶), -N(X⁶)(X⁶), - S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 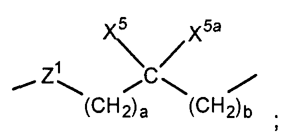
where a and b are each independently 0, 1, 2 or 3;
X⁵ and X^{5a} are each independently selected from the group consisting of hydrogen, CF₃, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of formula I;
E is -O-, -S-, -CH=CH- or an aromatic moiety selected from 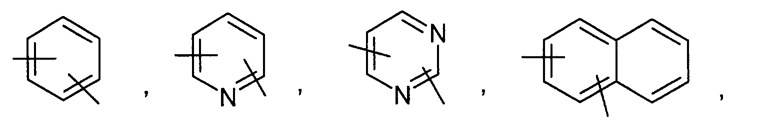 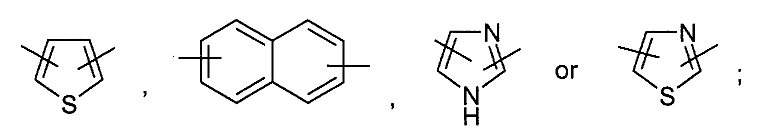 said aromatic moiety in the definition of E optionally substituted with up to three halo, hydroxy, -N(R^{c})(R^{c}), (C₁-C₆)alkyl or (C₁-C₆)alkoxy;
R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
R^{c}, for each occurrence, is independently hydrogen or (C₁-C₆)alkyl; a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substituents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl,-S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3.

3. A use according to claim 2 wherein said GHS is a compound of the formula 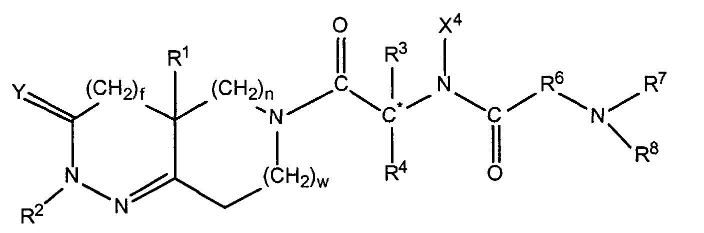 a racemic-diastereomeric mixture or optical isomer of said compound or a pharmaceutically-acceptable salt or prodrug thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl,-(C₁-C₅)alkyl-X¹-(C₀-C₅)alkyl-A¹ or -(C₁-C₅)alkyl-X¹-(C₁-C₅)alkyl-(C₃-C₇)cycloalkyl;
where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 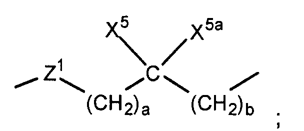
where a and b are independently 0, 1, 2 or 3;
X⁵ and X^{5a} are each independently selected from the group consisting of hydrogen, trifluoromethyl, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
X⁷ is hydrogen or (C₁-C₆)alkyl optionally substituted with hydroxyl; and
m for each occurrence is independently 0, 1 or 2;
with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

4. A use according to claim 3 wherein the GHS is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of said GHS or said prodrug.

5. A use according to claim 4 wherein the GHS is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate.

6. A combination comprising a GHS, a prodrug thereof or a pharmaceutically acceptable salt thereof or of said prodrug and a second therapeutic agent comprising a recombinant growth hormone or an additional GHS selected from the group consisting of GHRP-6, GHRP-1, GHRP-2, growth hormone releasing factor and an analog of growth hormone releasing factor.

7. A combination comprising a GHS, a prodrug thereof or pharmaceutically acceptable salt thereof or of said prodrug and a second therapeutic agent selected from methotrexate, dapsone, a glucocorticoid or an antimalarial, a prodrug of said agent or a pharmaceutically acceptable salt thereof or of said prodrug.

8. A combination according to claim 7 wherein said glucocorticoid is prednisone, betamethasone dipropionate, clobetasol, diflorasone diacetate, halobetasol propionate, amcinonide, desoximetasone, fluocinonide, halcinonide, betamethasone valerate, triamcinolone acetate, fluocinolone acetonide, flurandrenolide, hydrocortisone valerate, triamcinolone acetonide, hydrocortisone butyrate, alclometasone dipropionate, desonide, mometasone furoate, dexamethasone, hydrocortisone or methylprednisolone acetate.

9. A combination according to claim 7 or claim 8 wherein said antimalarial is chloroquine, hydroxychloroquine, quinacrine or quinine.

10. A combination comprising a GHS, a prodrug thereof or pharmaceutically acceptable salt thereof or of said prodrug and a second therapeutic agent selected from prednisone, sulfasalazine, mesalamine and olsalazine, a prodrug thereof or a pharmaceutically acceptable salt of said agent or said prodrug and a pharmaceutically acceptable carrier, vehicle or diluent.

11. A combination according to any one of claims 7 to 10 wherein said GHS is a compound of the Formula I: 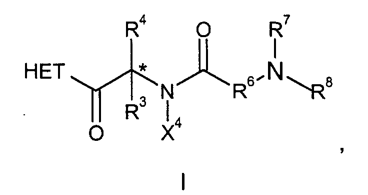 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug,
wherein:
HET is a heterocyclic moiety selected from the group consisting of 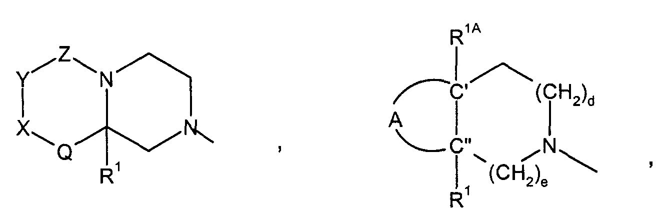 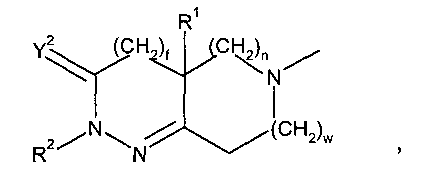 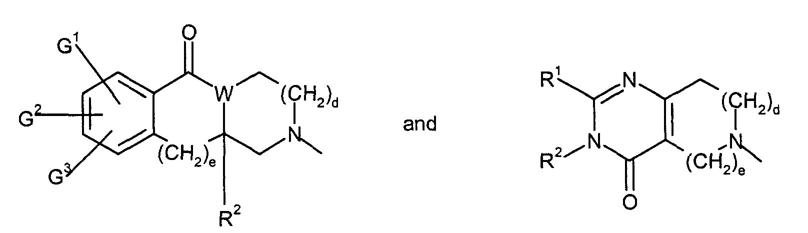
d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
-NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino, -(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(OH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, (CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxy, -C(O)OX⁶, -C(O)N(X⁶)(X⁶), -N(X⁶)(X⁶),-S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 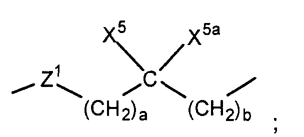
where a and b are each independently 0, 1, 2 or 3;
X⁵ and X^{5a} are each independently selected from the group consisting of hydrogen, CF₃, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of formula I;
E is -O-, -S-, -CH=CH- or an aromatic moiety selected from 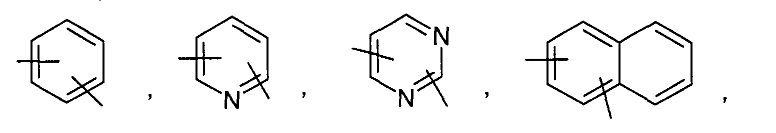 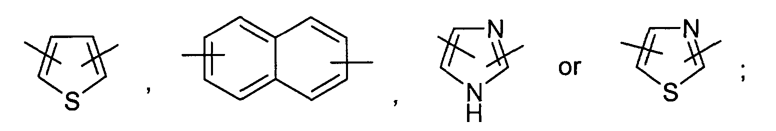 said aromatic moiety in the definition of E optionally substituted with up to three halo, hydroxy, -N(R^{c})(R^{c}), (C₁-C₆)alkyl or (C₁-C₆)alkoxy;
R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
R^{c}, for each occurrence, is independently hydrogen or (C₁-C₆)alkyl;
a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substituents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4-to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl,-S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3.

12. A combination according to claim 11 wherein said GHS is a compound of the formula 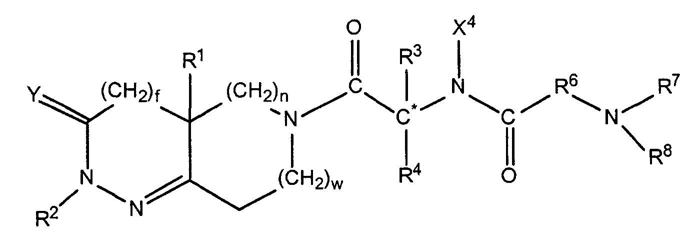 a racemic-diastereomeric mixture or optical isomer of said compound or a pharmaceutically-acceptable salt and prodrug thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)qN(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl,-(C₁-C₅)alkyl-X¹-(C₀-C₅)alkyl-A¹ or -(C₁-C₅)alkyl-X¹-(C₁-C₅)alkyl-(C₃-C₇)cycloalkyl;
where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 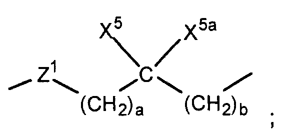
where a and b are independently 0, 1, 2 or 3;
X⁵ and X^{5a} are each independently selected from the group consisting of hydrogen, trifluoromethyl, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
X⁷ is hydrogen or (C₁-C₆)alkyl optionally substituted with hydroxyl; and m for each occurrence is independently 0, 1 or 2;
with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

13. A combination according to claim 12 wherein the GHS is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of said GHS or said prodrug.

14. A combination according to claim 13 wherein the GHS is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate.

15. A pharmaceutical composition comprising a combination according to any of claims 7 to 14 and a pharmaceutically acceptable carrier, vehicle or diluent.

16. Use of a combination according to any one of claims 7 to 14 for the manufacture of a medicament for separate, sequential or simultaneous administration of the GHS and the second therapeutic agent, for the treatment of systemic lupus erythematosus

17. A product containing:
a) a GHS, a prodrug thereof or a pharmaceutically acceptable salt of said GHS or said prodrug; and
b) a therapeutic agent selected from methotrexate, dapsone, a glucocorticoid or an antimalarial, a prodrug thereof or a pharmaceutically acceptable salt of said agent or said prodrug;
as a combined preparation for the treatment of systemic lupus erythematosus.

18. A product containing:
a) a GHS, a prodrug thereof or a pharmaceutically acceptable salt of said GHS or said prodrug; and
b) a therapeutic agent selected from prednisone, sulfasalazine, mesalamine and olsalazine, a prodrug thereof or a pharmaceutically acceptable salt of said agent or said prodrug;
as a combined preparation for the treatment of systemic lupus erythematosus.
